# EUROPEAN PATENT SPECIFICATION

(11) **EP 3 561 046 B1**
(45) Date of publication and mention of the grant of the patent: **30.06.2021**
(21) Application number: 19153202.7
(22) Date of filing: 23.01.2019
(51) Int. Cl.: C12M 3/10, C12M 1/16

(54) **DYNAMIC SOLID-STATE FERMENTATION APPARATUS**
DYNAMISCHE FESTKÖRPERFERMENTATIONSVORRICHTUNG
APPAREIL DE FERMENTATION À SEMI-CONDUCTEUR DYNAMIQUE

(30) Priority: 27.04.2018 IT 201800004911
(43) Date of publication of application: 30.10.2019
(73) Proprietor: Gibob S.R.L., 20142 Milano (IT)
(72) Inventor: GIUSEPPE, BOBBIESI, 20152 MILANO (IT)
(74) Representative: Forattini, Amelia

(56) References cited:
- WO-A1-2014/093444
- WO-A1-2015/011598
- CN-A- 102 816 704
- CN-U- 201 506 789
- CN-U- 201 737 943
- US-A- 2 091 850
- US-A- 5 427 947

## Description

The present invention relates to a dynamic solid-state fermentation apparatus, usable in particular to obtain products and semifinished products from microbiological processes of fermentation and/or sporification.

Since antiquity it is known to use fermentation processes that are set up by using solid raw materials, in conditions of controlled quantity of available water, currently known as SSF (solid-state fermentation).

SSF processes, originally used to produce particular types of traditional foods and beverages, have been used in more recent times in the microbiological production of biomasses and biomolecules, assuming an increasingly important position.

This has required the provision of appropriate apparatuses, better known as bioreactors, capable of providing an environment suitable for the propagation of biological microorganisms or for fermentation on the part thereof.

Science literature reports various studies and researches related to SSF, which mostly relate to laboratory activities performed by using small apparatuses, generally constituted by glass tubes with a diameter of a few centimeters, therefore in static conditions, with very low possibilities to control the various process parameters and using rudimentary culture media, which are naturally available and are not composed specifically.

At the industrial level, various types of bioreactors for SSF, both static and dynamic, have been tested; in particular, a first type is constituted by tray bioreactors, or bag system bioreactors, in which fermentation is performed by using a culture of microorganisms that is arranged on trays or more frequently introduced in bags on pre-sterilized substrates; the containers are then kept in stationary conditions, without mechanical agitation, at a controlled temperature and if possible controlled humidity.

These reactors clearly require a lot of space, labor and, in the second case, also a considerable consumption of bags.

Another type of reactor is the packed bed bioreactor, constituted by a vertically or horizontally extended chamber which has a forced ventilation system; humid air is in fact supplied from the bottom of the chamber and passes through the culture bed, with an intermittent flow.

A variation of packed bed reactor has, inside the chamber, a worm screw that mixes the culture at preset intervals.

Those bioreactors generally exhibit severe problems in the control of humidity, temperatures, and dissipation of the heat generated by fermentation as well as in performing the filling/emptying and cleaning operations.

A further type of reactor is a rotating drum bioreactor, which essentially consists of a large cylinder which is arranged horizontally and rotates at regular intervals; flow splitters can be provided inside the cylinder in order to improve the mixing produced by rotation.

In a variation of the rotating drum bioreactor, known as stirred drum bioreactor, the cylinder that contains the culture does not move and the mixing action is provided by a blade-based agitation system inside it.

There are also reactors in which, during fermentation, the culture is continuously kept in motion by means of an air flow combined with the action of adapted mechanical agitators; these are so-called continuously-mixed, forcefully-aerated bioreactors.

The greatest limitation of these reactors resides in that they tend to damage the culture medium and the culture itself, in particular in the case of some fungi, due to the shearing produced by mechanical agitators, in addition to the damage of an air flow that does not correspond to the flow that is useful for the biological process; moreover, they can be difficult or incomplete to unload.

A variation of the preceding reactors is constituted by an intermittently-mixed forcefully-aerated bioreactor, in which agitation is not continuous but is intermittent, with a consequent greater possibility of adaptation to the fermentation process and a lower breakup of the culture medium; however the intermittently-mixed forcefully-aerated bioreactor substantially has the same problems of the other prior art reactors.

Continuous solid-state fermentation bioreactors are also used in the industrial field in which essentially various mechanical instruments are used to produce continuous agitation in the culture bed; generally they have considerable limitations linked to the intensity of the stirring, which in addition to requiring a large amount of energy can damage the microorganisms involved in fermentation or their processes.

In addition to the above mentioned problems, it should be considered that few of the above-cited bioreactors are suitable to produce pure products based on microorganisms or secondary metabolites thereof or enzymes, with simultaneous complete control of sterility and of all process parameters, which experimental tests have proved to be necessary.

Almost all known systems are used for productions of products with a low technology content and a low commercial value, which are obtained in conditions that are scarcely controllable and reproducible (for example composting, etc.).

Also, in some cases, one speaks of SSF, but actually a complete fermentation process is not performed but only a part thereof is performed, i.e., sporification on surfaces also of culture powders, generally fungal cultures, obtained by means of submerged state bioreactors (SmF) and subsequently spread on solid bodies or powders, preferably with few nutrients or nutrients that are totally imbalanced in order to facilitate sporification, or also by reducing the water content, without however being able to control it.

It is also noted that no prior art bioreactor or system for obtaining even just sporification allows to perform processes downstream of the fermentation or sporification process in the same apparatus in which fermentation or sporification has occurred.

WO2015/011598 discloses an apparatus for the purification of wastewater having a cylindrical container delimiting a reaction chamber receiving water to be treated and a free body which has a surface for the formation of bacterial colonies capable of purifying the water. The container rotates for periodically driving the support of the free body between an immersed condition, wherein it is totally covered with water to be treated, and an emerged condition, wherein it emerges, at least partially, from the water to be treated.

CN201506798(U) discloses a solid-state aerobic fermentation device having a fermentor and a condensation jacket that is used for clamping the fermentor, wherein the fermentor has an upper seal head, a lower seal head and a fermentation chamber that is arranged between the upper seal head and the lower seal head; the bottom part of the fermentation chamber is provided with a piece of filter cloth and a wire mesh; and the fermentation chamber is internally provided with a temperature electrode used for measuring the temperature of the fermentation chamber.

CN102816704 discloses a medium inoculation method and apparatus in the Cordyceps cicadae solid fermentation industry, wherein cleaned wheat is conveyed into an integrated tank and subjected to stewing and sterilization; the wheat is then cooled by a cooling device and a bacterial solution is introduced into the tank, which rotates around a shaft to make the wheat and the bacterial solution fully and uniformly mixed.

CN201737943(U) discloses a rotary barrel type solid-state fermentation equipment having a fermentation tank wherein humidifying and mixing, stewing and sterilizing, temperature reducing, inoculating, temperature-controlled fermenting and discharging of the materials are performed in the same fermentation tank.

The aim of the present invention is to provide an apparatus for dynamic solid-state fermentation that overcomes the drawbacks of the cited prior art.

Within the scope of this aim, a particular object of the invention is to provide an apparatus that allows to optimize an SSF fermentation process by creating the best necessary conditions, also by means of a movement system of a specific type.

A further object of the invention is to provide an apparatus that allows to keep under complete control substantially all the parameters involved in the fermentation process.

A further object of the invention is to provide an apparatus that can be used not only for fermentation or for sporification alone but also to perform the subsequent processing steps downstream of these processes, without extracting the fermented medium, in order to obtain semifinished or finished formulations in various physical forms.

A further object of the invention is to provide an apparatus that allows to obtain total homogeneity of the material treated inside it.

A further object of the invention is to provide an apparatus that has relatively low management costs, also by virtue of the complete automation and minimization of the required operations.

This aim, these objects and others which will become better apparent hereinafter are achieved by a dynamic solid-state fermentation apparatus, comprising at least one container forming a sterilizable compartment for containing and mixing a solid or semisolid material; said container comprising at least one first port for accessing said sterilizable compartment; said container being associated to a supporting structure and rotating about an axis of rotation; said apparatus comprising fermentation control means for controlling a fermentation process of said material and downstream processing means processing said material after said fermentation process, said downstream processing means been removably associated with said container by coupling means; said downstream processing means interacting with said material directly inside said container producing semifinished products and finished products; said apparatus being characterized in that said container comprises a central hollow body having a substantially cylindrical shape; said central body being closed at the mutually opposite ends by two peripheral hollow bodies which have an asymmetric substantially frustum-like shape; each of said peripheral bodies comprising at least one generatrix that is substantially parallel to at least one corresponding generatrix of said central body and the remaining generatrices being inclined with respect to the corresponding generatrices of said central body; in said two peripheral bodies said two generatrices that are substantially parallel to at least one generatrix of said central body being respectively arranged in mutually diametrically opposite positions with respect to the axis of symmetry of said central body.

Further characteristics and advantages will become better apparent from the description of a preferred but not exclusive embodiment of a dynamic solid-state fermentation apparatus according to the invention, illustrated by way of non-limiting example in the accompanying drawings, wherein:

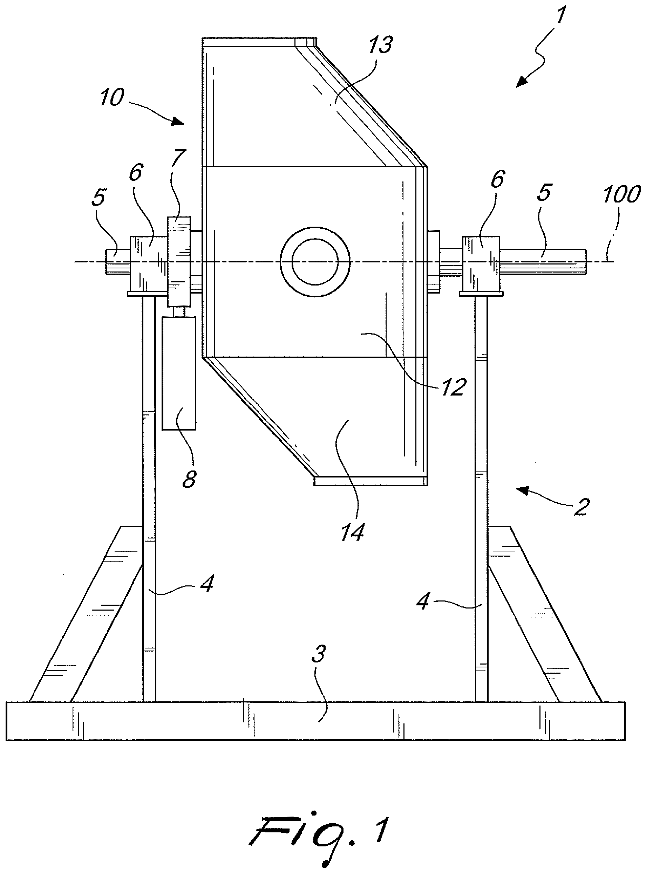

Figure 1 is a front view of an apparatus according to the invention;

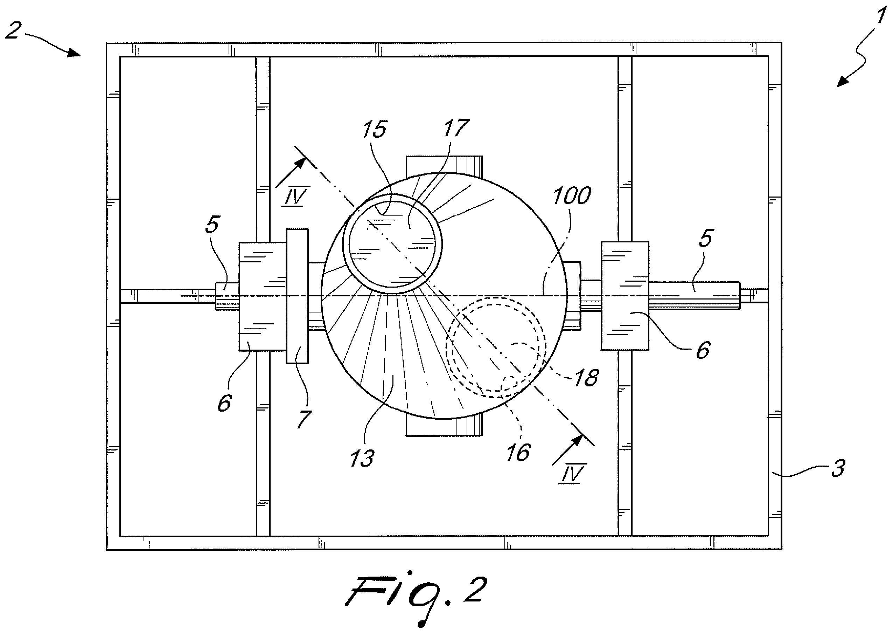

Figure 2 is a plan view of the apparatus according to the invention;

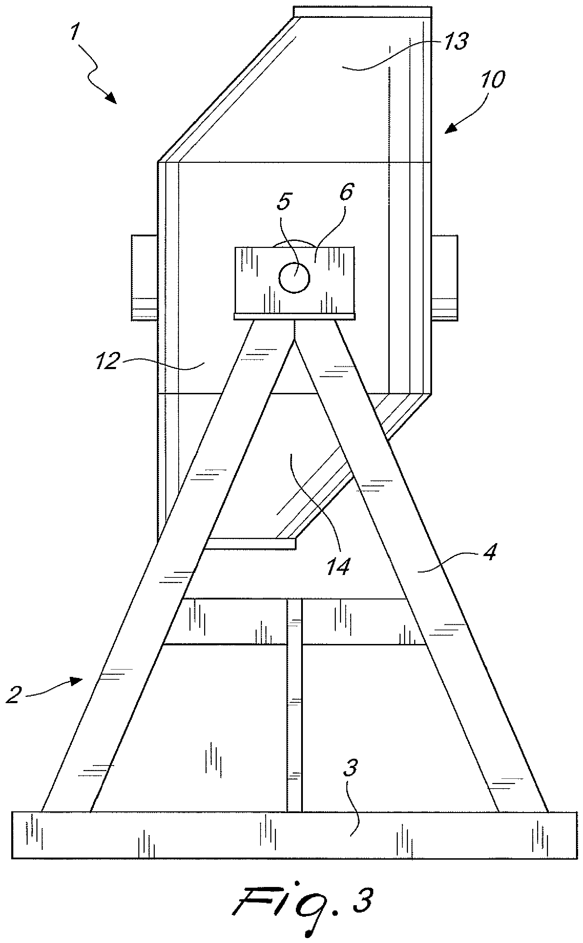

Figure 3 is a side view of the apparatus according to the invention;

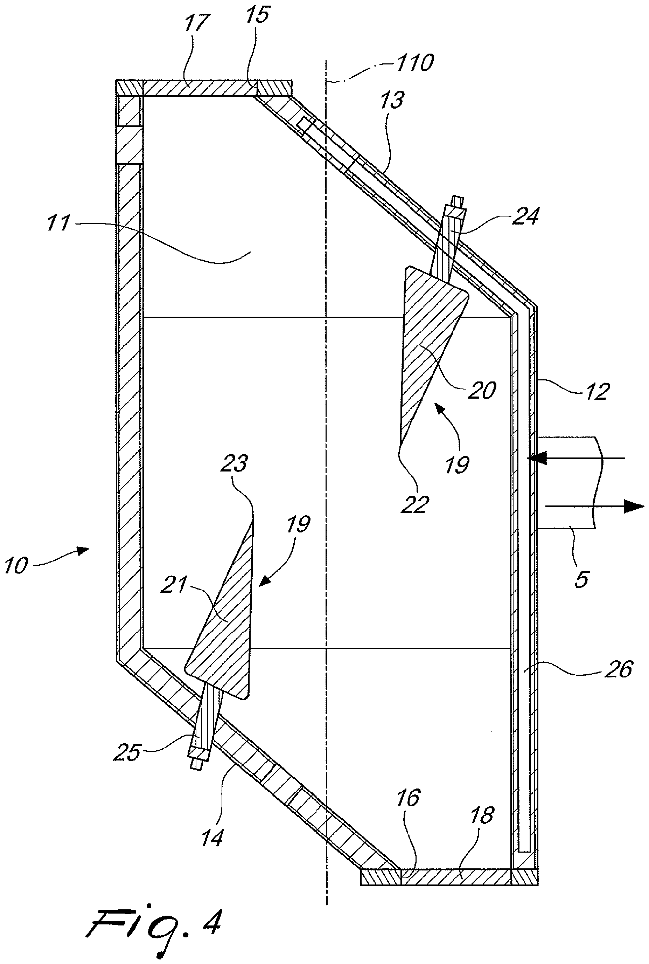

Figure 4 is a sectional view, taken along the plane IV-IV of Figure 2;
Figures 5 to 9 are schematic views of a succession of rotation steps of the container of the apparatus according to the invention, during a rotation of substantially 180° and with a filling coefficient of the container equal to approximately 50%, and the consequent movement of the material undergoing fermentation inside it.

With reference to the cited figures, the dynamic solid-state fermentation apparatus is generally designated by the reference numeral 1 and includes a container 10 which is rotatably connected to a supporting structure 2, about a rotation axis 100.

The container 10 internally defines a sterilizable compartment 11 which, for example, can be sterilized by using steam and is adapted to contain and mix a solid or semisolid material 50; in the specific case, the material 50 is constituted by a culture medium undergoing sterilization or fermentation.

In the illustrated embodiment, the supporting structure 2 includes a base 3 from which two uprights 4 rise and support the container 10 by means of hollow axle shafts 5 supported by bearings 6.

According to the present invention, the apparatus 1 includes fermentation control means, for controlling the fermentation process of the material 50, and downstream processing means that treat the material 50 directly in the container 10, once fermentation has ended.

The special shape of the container 10 allows to optimize the fermentation process, substantially controlling all of its parameters, and performing processes downstream of the fermentation process directly in the container 10.

The container 10 includes a central body 12, which is formed by an internally hollow cylinder, which is closed at the opposite ends by two peripheral bodies 13 and 14 which are substantially frustum-shaped and internally hollow.

Each peripheral body 13, 14 has a generatrix that is substantially parallel to a corresponding generatrix of the central body 12, in such a manner as to constitute in practice a continuation thereof, and the remaining generatrices are inclined with respect to the corresponding generatrices of the central body 12.

In practice the peripheral bodies 13 and 14 are shaped like oblique frustums, as clearly shown in the accompanying figures.

The two generatrices which in the peripheral bodies 13 and 14 are parallel to corresponding generatrices of the central body 12 are arranged in diametrically mutually opposite positions with respect to the axis of symmetry 110 of the central body 12.

In a preferred embodiment of the present invention, the height of the central body 12 is equal to approximately 9/10 of its diameter; each peripheral body 13, 14 has a larger end face with a diameter that substantially corresponds to the diameter of the central body 12, a smaller end face with a diameter equal to approximately 3/10 of the diameter of the central body 12, and a height equal to approximately 6/10 of the diameter of the central body 12.

The volume of the container 10 is substantially equal to approximately the cube of the diameter of the central body 12.

However, it is evident that different embodiments of the invention might provide, for the container 10, geometries and dimensions that are different from the ones indicated above by way of example.

The container 10 is preferably made of metallic material, for example stainless steel.

Advantageously, the container 10 has a jacket 26 wherein a fluid circulates at a controlled temperature allowing a thermostatic control of the container 10.

The fluid, such as steam, is circulated inside the jacket 26 by means of a four way rotating joint associated with the shaft 5.

The container 10 includes a first port 15 and preferably also a second port 16 for access to the sterilizable compartment 11 in order to allow the feeding and discharge of the material 50 and of any adjuvants of the fermentation process.

Advantageously, the first port 15 and the second port 16 are formed at the smaller end faces of the peripheral bodies 13 and 14 and are closed by hermetic lids, respectively designated by the reference numerals 17 and 18, which are mounted on an adapted coupling means, constituted in the specific case by a pair of flanges.

Discharge valves can be installed on the flanges as a replacement of the hermetic lids 17 and 18.

On the walls of the container 10 can have one or more viewports, sterilizable sampling ports, couplings for probes and sensors of various types (also suitable for wireless transmission of collected data), and other provisions which are already per se known and are therefore not described.

An anti-aggregation device 19 is installed inside the container 10, in the sterilizable compartment 11, and is designed to provide a first coarse breakup of any compact blocks of material 50 and to improve the mixing produced by rotation, also avoiding the content undergoing fermentation from compacting.

In a preferred embodiment of the present invention, the anti-aggregation device 19 includes a pair of blades 20 and 21 which are detachably associated with the internal walls of the peripheral bodies 13 and 14.

Each blade 20, 21 has a tip, designated respectively by 22 and 23, which is directed toward the smaller end face of the peripheral body 13, 14 that lies opposite the one with which it is associated.

In practice, assuming that the blade 20 is associated with the peripheral body 13, its tip 22 is directed toward the smaller end face of the peripheral body 14; the tip 23 of the blade 21 is instead directed toward the smaller end face of the peripheral body 13.

Preferably, each blade 20, 21 is inclined by an angle comprised between 10° and 90° and even more preferably by an angle comprised between 20° and 25° with respect to the generatrices of the central body 12.

In any case, the position of the blades 20 and 21 and their inclination is adjustable from the outside by means of fastener and adjustment means, designated respectively by 24 and 25.

The downstream processing means include devices for breaking up and/or milling the material 50, devices for extraction with extraction agents, homogenization devices with fluids until solid particles of very small size are obtained, as well as other devices used to obtain semifinished products or finished formulations, in various physical forms; these devices are not described herein since they are substantially conventional and known to the person skilled in the art.

Advantageously, the downstream processing means can be detachably associated with the container 10 by coupling means otherwise used to retain the hermetic lids 17 and 18.

The container 10 rotates about the rotation axis 100, which is preferably inclined by an angle equal to approximately 45° with respect to the straight line that traces the minimum distance between the two generatrices which, in the peripheral bodies 13 and 14, are respectively parallel to the generatrices of the central body 12.

The rotation axis 100 is defined by the hollow axle shafts 5, which can rest on load cells, not shown in the figures, and allow to detect any weight variations that have occurred in the material 50 following additions/losses of water or of other substances.

The hollow axle shafts 5 are fixed to the container 10 and are functionally connected to a rotary actuation means.

In the specific case, the rotary actuation means includes a gearmotor 7 actuated by a motor 8 driven by means of an inverter, so as to be able to vary the rotation rate of the vessel 10 over a rather broad range, preferably comprised between 1 and 60 rotations per hour.

The rotary actuation means also allows to reverse the direction of rotation of the container 10, to induce oscillating motions thereof, to stop and restart its motion, and others.

Advantageously, the hollow axle shafts 5 are provided with hermetic rotary joints which allow their connection to pipes of distribution circuits, which are not shown in the accompanying figures.

These circuits allow for example the circulation of a thermostatic control fluid in the interspace of the container 10, the feeding of steam into the sterilizable compartment 11 and in the interspace, the injection of gases into the sterilizable compartment 11, the extraction of gases from the sterilizable compartment 11 (until vacuum is produced), the introduction in the sterilizable compartment 11 of substances (feeds) adapted to feed the fermentation of the material 50, and the introduction in the sterilizable compartment 11 of liquid substances of various kinds, including inoculi and water.

Advantageously, at least part of the pipes that are connected to the sterilizable compartment 11 are extractable, in order to allow to mount on their end that leads into the sterilizable compartment 11 an adapted means for modifying the flow, such as for example nozzles for distributing fluids appropriately, or for avoiding the leakage of substances (powders, spores, microorganisms, etc.), such as for example absolute filtration cartridges, and others.

Adapted adjustment elements, such as valves, controlled by an adapted actuation means, and multiple sensors for detecting the various process parameters, such as for example temperature probes, pressure probes, relative humidity probes, pH probes, oxygen probes, etc., are also mounted in the distribution circuits.

Advantageously, the sensors can be preset for the transmission, even wirelessly, of the collected data to an electronic unit.

An electronic unit, not shown in the accompanying figures, is in fact provided in the apparatus 1, is controlled by software and can be controlled locally by virtue of an adapted user interface means or remotely.

In practice, the electronic unit allows to control the entire fermentation process of the material 50 as well as all the subsequent operations performed downstream of the fermentation process.

The electronic unit reads the values detected by the sensors with which the apparatus 1 is equipped and, on the basis of the settings received from the user, accordingly controls substantially all the operating elements that are present, including in particular the valve actuation means, the rotary actuation means and any means for processing downstream of the fermentation process.

The operation of the apparatus according to the invention is evident from what has been described and illustrated.

In practice, the material 50 is introduced in the sterilizable compartment 11 formed by the container 10, for example through the first mouth 15.

The material 50 is then steam sterilized to be then cooled and inoculated.

The container is then turned by the gearmotor 7 actuated by the motor 8, so that the mixing of the material 50 and its breakup by the blades 20 and 21 can begin.

During the rotation of the container 10, or during a machine downtime, the fermentation air and all the substances required to cause the fermentation of the material 50 are introduced in the sterilizable compartment 11, simultaneously recreating the optimum environment for the fermentation process.

For this purpose, the electronic unit reads the values acquired by the various sensors (temperature, relative humidity, pH, pressure, oxygen, etc.) and, on the basis of the settings received from the user, controls accordingly substantially all the operating elements that are present, including in particular the valve actuation means and the rotary actuation means.

Once the fermentation process has been completed, the fermented material can be dried and subsequently, by virtue of the downstream processing means, it is possible to perform on the material 50 the additional processing steps provided downstream of the process without extracting the material from the container 10.

When the material 50 has been completely transformed into the desired semifinished products or finished formulations, in various physical forms, it is extracted from the container 10, for example via the second port 16, or by means of the discharge valve mounted on the hermetic lid 18.

The type of motion that determines the mixing of the material 50 inside the container 10, which is necessary in order to optimize the fermentation process and to have full homogeneity of the material 50, is determined mainly by the shape of the internal walls of the latter and by the blades 20 and 21.

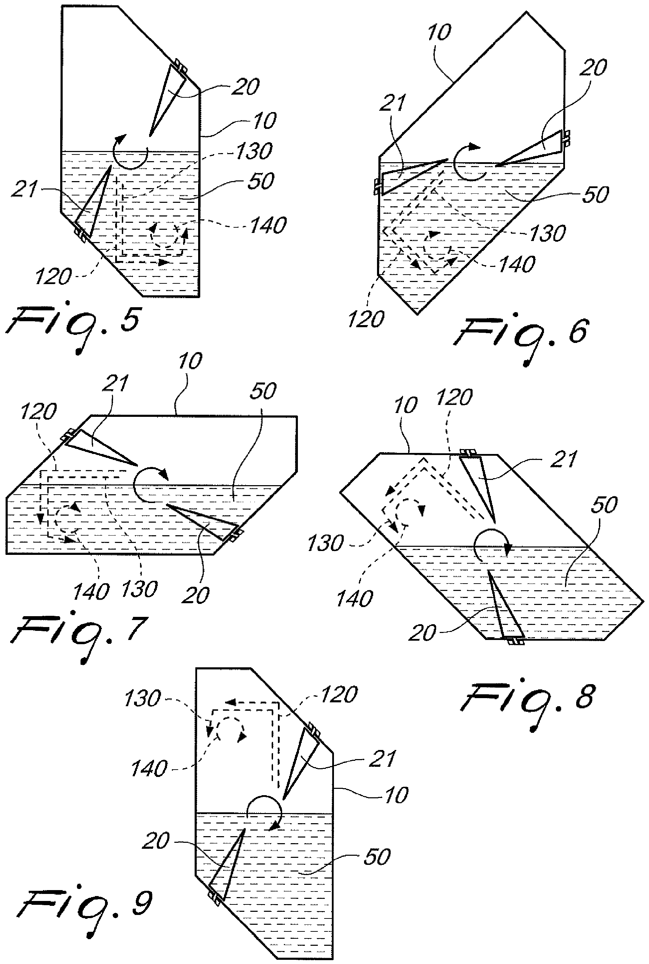

In particular, the motion of the material 50 is substantially the result of various components that are designated schematically in Figures 5 to 9 with dashed arrows, designated respectively by 120, 130 and 140.

In practice it has been found that the invention achieves the intended aim and objects, an apparatus for dynamic solid-state fermentation having been provided which is equipped with an innovative container and adapted devices by means of which it is possible to maintain constant control of the concentrations of available water, of gas (as oxygen, dry air, humid air, nitrogen, vacuum), of the temperature, of the relative humidity, of the pH and of the mixing speed of its contents.

This allows to optimize SSF fermentation processes, creating the best conditions required for the culture medium, for the stock being used and for the desired products.

The apparatus according to the present invention can be used not only for fermentation or for sporification alone but also to perform the subsequent steps for processing downstream of the processes, without extracting and moving in other apparatuses the fermented material from the container.

The materials used, so long as they are compatible with the specific use, as well as the contingent shapes and dimensions, may of course vary according to the requirements and the state of the art.

## Claims

1. A dynamic solid-state fermentation apparatus, comprising at least one container **(10)** forming a sterilizable compartment **(11)** for containing and mixing a solid or semisolid material **(50);** said container **(10)** comprising at least one first port **(15)** for accessing said sterilizable compartment **(11);** said container **(10)** being associated to a supporting structure **(2)** and rotating about an axis of rotation **(100);** said apparatus comprising fermentation control means for controlling a fermentation process of said material **(50)** and downstream processing means processing said material **(50)** after said fermentation process, said downstream processing means been removably associated with said container **(10)** by coupling means; said downstream processing means interacting with said material **(50)** directly inside said container **(10)** producing semifinished products and finished products; said apparatus being **characterized in that** said container **(10)** comprises a central hollow body **(12)** having a substantially cylindrical shape; **said central body (12) having an axis of symmetry (110) which is substantially perpendicular to said axis of rotation (100);** said central body **(12)** being closed at the mutually opposite ends by two peripheral hollow bodies **(13, 14)** which have an asymmetric substantially frustum-like shape; each of said peripheral bodies **(13, 14)** comprising at least one generatrix that is substantially parallel to at least one corresponding generatrix of said central body **(12)** and the remaining generatrices being inclined with respect to the corresponding generatrices of said central body **(12);** in said two peripheral bodies **(13, 14)** said two generatrices that are substantially parallel to at least one generatrix of said central body **(12)** being respectively arranged in mutually diametrically opposite positions with respect to the axis of symmetry **(110)** of said central body **(12).**

2. The apparatus according to claim 1, **characterized in that** each one of said peripheral bodies **(13, 14)** comprises a larger end face with a diameter that substantially corresponds to the diameter of said central body **(12)** and a smaller end face with a diameter that is preferably approximately 3/10 of the diameter of said central body **(12);** the height of each of said peripheral bodies **(13, 14)** being preferably approximately 6/10 of the diameter of said central body **(12);** the height of said central body **(12)** being preferably approximately 9/10 of its diameter; the volume of said container **(10)** being substantially equal to approximately the cube of said diameter.

3. The apparatus according to claim 1, **characterized in that** said axis of rotation **(100)** of said container **(10)** is inclined at an angle equal to approximately 45° with respect to the straight line that traces the shortest distance between the generatrices of said peripheral bodies **(13, 14)** that are parallel to at least one generatrix of said central body **(12).**

4. The apparatus according to claim 1, **characterized in that** it comprises at least one anti-aggregation device **(19)** arranged inside said sterilizable container **(10);** said anti-aggregation device **(19)** comprising one or more blades **(20, 21)** that are removably associated with the inner walls of said peripheral bodies **(13, 14);** each one of said blades **(20, 21)** having a tip **(22, 23)** directed toward the smaller end face of the peripheral body **(13)** that is opposite the one with which it is associated.

5. The apparatus according to claim 4, **characterized in that** said one or more blades **(20, 21)** that are removably associated with the inner walls of said peripheral bodies **(13),** with the possibility of adjusting the position and inclination; each of said blades **(20, 21)** being inclined by an angle between approximately 10° and approximately 90° with respect to the generatrices of said central body **(12).**

6. The apparatus according to claim 4, **characterized in that** said one or more blades **(20, 21)** that are removably associated with the inner walls of said peripheral bodies **(13),** with the possibility of adjusting the position and inclination; each of said blades **(20, 21)** being inclined by an angle between approximately 20° and approximately 25°, with respect to the generatrices of said central body **(12).**

7. The apparatus according to claim 1, **characterized in that** said downstream processing means comprises one or more milling devices removably associated with said container **(10),** one or more homogenization devices removably associated with said container **(10),** one or more extraction devices removably associated with said container **(10),** and one or more discharge valves for solids or liquids; said downstream processing means being arranged substantially at the smaller end faces of said peripheral bodies **(13)** and being associable with said container **(10)** by said coupling means.

8. The apparatus according to claim 1, **characterized in that** said container **(10)** is rotatably associated with said supporting structure **(2)** by means of at least one hollow axle shaft **(5),** which is functionally connected to a rotary actuation means **(7, 8);** said rotating actuation means **(7, 8)** being controlled by said fermentation control means.

9. The apparatus according to claim 8, **characterized in that** said container **(10)** has a jacket **(26)** wherein a fluid circulates at a controlled temperature allowing a thermostatic control of the container **(10).**

10. The apparatus according to claim 1, **characterized in that** said fermentation control means comprises a plurality of sensors for detecting the process parameters associated with said container **(10);** at least part of said sensors being functionally connected to a data transmission means.

11. The apparatus according to claims 8 and 11, **characterized in that** said fermentation control means comprises at least one electronic unit provided with a user interface means for on-site and remote control; said at least one electronic unit being functionally connected to said sensors, to said downstream processing means and to said rotary actuation means.

## Patentansprüche

1. Eine dynamische Festkörperfermentationsvorrichtung, die mindestens einen Behälter (10) umfasst, der eine storilisierbare Kammer (11) zur Aufnahme und zum Mischen eines festen oder halbfesten Materials (50) bildet; wobei der Behälter (10) mindestens eine erste Öffnung (15) für den Zugang zu der sterilisierbaren Kammer (11) umfasst; wobei der Behälter (10) mit einer tragenden Struktur (2)verbunden ist und sich um eine Drehachse (100) dreht; wobei die Vorrichtung Fermentationssteuerungsmittel zum Steuern eines Fermentationsprozesses des Materials (50) und stromabwärts angeordnete Verarbeitungsmittel umfasst, die das Material (50) nach dem Fermentationsprozess verarbeiten, wobei die stromabwärts angeordneten Verarbeitungsmittel über Kopplungsmittel lösbar mit dem Behälter (10) verbunden sind; wobei die stromabwärts angeordneten Verarbeitungsmittel mit dem Material (50) direkt innerhalb des Behälters (10) zusammenwirken und so halbfertige und fertige Produkte erzeugen; wobei die Vorrichtung **dadurch gekennzeichnet ist, dass** der Behälter (10) einen zentralen Hohlkörper (12) mit einer im Wesentlichen zylindrischen Form umfasst; wobei der zentrale Körper (12) eine Symmetrieachse (110) hat, die im Wesentlichen senkrecht zu der Drehachse (100) ist; wobei der zentrale Körper (12) an den einander gegenüberliegenden Enden durch zwei periphere Hohlkörper (13, 14) verschlossen ist, die im Wesentlichen eine asymmetrische Kegelstumpfform haben; wobei jeder der peripheren Körper (13, 14) mindestens eine Erzeugende umfasst, die im Wesentlichen parallel zu mindestens einer entsprechenden Erzeugenden des zentralen Körpers (12) ist, und die restlichen Erzeugenden im Verhältnis zu den entsprechenden Erzeugenden des zentralen Körpers (12) geneigt sind; wobei in den zwei peripheren Körpern (13, 14) die beiden Erzeugenden, die im Wesentlichen parallel zu mindestens einer Erzeugenden des zentralen Körpers (12) sind, entsprechend in einander diametral gegenüberliegenden Positionen mit Bezug auf die Symmetrieachse (110) des zentralen Körpers (12) angeordnet sind.

2. Die Vorrichtung gemäß Anspruch 1, **dadurch gekennzeichnet, dass** jeder der peripheren Körper (13, 14) eine größere Stirnseite mit einem Durchmesser umfasst, der im Wesentlichen dem Durchmesser des zentralen Körpers (12) entspricht, und eine kleinere Stirnseite mit einem Durchmesser, der vorzugsweise ungefähr 3/10 des Durchmessers des zentralen Körpers (12) beträgt; wobei die Höhe jedes der peripheren Körper (13, 14) vorzugsweise ungefähr 6/10 des Durchmessers des zentralen Körpers (12) beträgt; wobei die Höhe des zentralen Körpers (12) vorzugsweise ungefähr 9/10 seines Durchmessers beträgt; wobei das Volumen des Behälters (10) im Wesentlichen ungefähr gleich der dritten Potenz des Durchmessers ist.

3. Die Vorrichtung gemäß Anspruch 1, **dadurch gekennzeichnet, dass** die Drehachse (100) des Behälters (10) in einem Winkel ungefähr gleich 45° mit Bezug auf die gerade Linie geneigt ist, die den kürzesten Abstand zwischen den Erzeugenden der peripheren Körper (13, 14) darstellt, die parallel zu mindestens einer Erzeugenden des zentralen Körpers (12) sind.

4. Die Vorrichtung gemäß Anspruch 1, **dadurch gekennzeichnet, dass** sie mindestens eine Anti-Aggregationsvorrichtung (19) umfasst, die innerhalb des sterilisierbaren Behälters (10) angebracht ist; wobei die Anti-Aggregationsvorrichtung (19) ein oder mehrere Blätter (20, 21) umfasst, die lösbar mit den Innenwänden der peripheren Körper (13, 14) verbunden sind; wobei jedes der Blätter (20, 21) eine Spitze (22, 23) hat, welche zur kleineren Stirnseite des peripheren Körpers (13) hin weist, die derjenigen, mit welcher es verbunden ist, gegenüberliegt.

5. Die Vorrichtung gemäß Anspruch 4, **dadurch gekennzeichnet, dass** die einen oder mehreren Blätter (20, 21), die lösbar mit den Innenwänden der peripheren Körper (13) verbunden sind, mit der Möglichkeit, die Position und Neigung anzupassen; wobei jedes der Blätter (20, 21) in einem Winkel zwischen ungefähr 10° und ungefähr 90° mit Bezug auf die Erzeugenden des zentralen Körpers (12) geneigt ist.

6. Die Vorrichtung gemäß Anspruch 4, **dadurch gekennzeichnet, dass** die einen oder mehreren Blätter (20, 21), die lösbar mit den Innenwänden der peripheren Körper (13) verbunden sind, mit der Möglichkeit, die Position und Neigung anzupassen; wobei jedes der Blätter (20, 21) in einem Winkel zwischen ungefähr 20° und ungefähr 25° mit Bezug auf die Erzeugenden des zentralen Körpers (12) geneigt ist.

7. Die Vorrichtung gemäß Anspruch 1, **dadurch gekennzeichnet, dass** die stromabwärts angeordneten Verarbeitungsmittel Folgendes umfassen: eine oder mehrere Zerkleinerungsvorrichtungen, die lösbar mit dem Behälter (10) verbunden sind, eine oder mehrere Homogenisierungsvorrichtungen, die lösbar mit dem Behälter (10) verbunden sind, eine oder mehrere Extraktionsvorrichtungen, die lösbar mit dem Behälter (10) verbunden sind, und ein oder mehrere Auslassventile für feste oder flüssige Stoffe; wobei die stromabwärts angeordneten Verarbeitungsmittel im Wesentlichen an den kleineren Stirnseiten der peripheren Körper (13) angeordnet und über die Kopplungsmittel mit dem Behälter (10) verbindbar sind.

8. Die Vorrichtung gemäß Anspruch 1, **dadurch gekennzeichnet, dass** der Behälter (10) mit der tragenden Struktur (2) drehbar über mindestens einen hohlen Achsschaft (5) verbunden ist, welcher funktional mit einem Drehantriebsmittel (7, 8) verknüpft ist; wobei das Drehantriebsmittel (7, 8) von den Fermentationssteuerungsmitteln gesteuert wird.

9. Die Vorrichtung gemäß Anspruch 8, **dadurch gekennzeichnet, dass** der Behälter (10) einen Mantel (26) hat, worin ein Fluid mit einer kontrollierten Temperatur zirkuliert, was eine thermostatische Steuerung des Behälters (10) ermöglicht.

10. Die Vorrichtung gemäß Anspruch 1, **dadurch gekennzeichnet, dass** das Fermentationssteuerungsmittel eine Vielzahl von Sensoren zur Erfassung der mit dem Behälter (10) verknüpften Prozessparameter umfasst; wobei mindestens ein Teil der Sensoren funktional mit einem Datenübertragungsmittel verbunden ist.

11. Die Vorrichtung gemäß den Ansprüchen 8 und 11, **dadurch gekennzeichnet, dass** das Fermentationssteuerungsmittel mindestens eine Elektronikeinheit, ausgestattet mit einem Benutzeroberflächenmittel für die Steuerung vor Ort und die Fernbedienung umfasst; wobei die mindestens eine Elektronikeinheit funktional mit den Sensoren, den stromabwärts angeordneten Verarbeitungsmitteln und mit den Drehantriebsmitteln verbunden ist.

## Revendications

1. Appareil de fermentation à semi-conducteur dynamique, comprenant au moins un récipient (10) formant un compartiment stérilisable (11) permettant de contenir et de mélanger un matériau solide ou semi-solide (50); ledit récipient (10) comprenant au moins un premier orifice (15) permettant d'accéder audit compartiment stérilisable (11); ledit récipient (10) étant associé à une structure de support (2) et tournant autour d'un axe de rotation (100); ledit appareil comprenant des moyens de contrôle de fermentation permettant de commander un processus de fermentation dudit matériau (50) et des moyens de traitement aval traitant ledit matériau (50) après ledit processus de fermentation, lesdits moyens de traitement aval sont associés de manière amovible audit récipient (10) par des moyens de couplage; lesdits moyens de traitement aval interagissant avec ledit matériau (50) directement à l'intérieur dudit récipient (10) produisant des produits semi-finis et des produits finis; ledit appareil étant **caractérisé en ce que** ledit récipient (10) comprend un corps creux central (12) ayant une forme essentiellement cylindrique; ledit corps central (12) comportant un axe de symétrie (110) qui est essentiellement perpendiculaire audit axe de rotation (100); ledit corps central (12) étant fermé à des extrémités mutuellement opposées au moyen de deux corps creux périphériques (13, 14) qui ont une forme asymétrique essentiellement tronconique; chacun desdits corps périphériques (13, 14) comprenant au moins une génératrice qui est essentiellement parallèle à au moins une génératrice correspondante dudit corps central (12) et les génératrices restantes étant inclinées par rapport aux génératrices correspondantes du corps central (12); dans lesdits deux corps périphériques (13, 14) les deux génératrices essentiellement parallèles à au moins une génératrice dudit corps centrale (12) étant respectivement agencées dans des positions mutuellement diamétralement opposées par rapport à l'axe de symétrie (110) dudit corps central (12).

2. Appareil selon la revendication 1, **caractérisé en ce que** chacun desdits corps périphériques (13, 14) comprend une face d'extrémité plus grande avec un diamètre qui correspond essentiellement au diamètre dudit corps central (12) et une face d'extrémité plus petite avec un diamètre qui représente de préférence approximativement 3/10 du diamètre dudit corps central (12); la hauteur de chacun desdits corps périphériques (13, 14) représentant de préférence approximativement 6/10 du diamètre dudit corps central (12); la hauteur dudit corps central (12) représentant de préférence approximativement 9/10 de son diamètre; le volume dudit récipient (10) étant essentiellement égal à approximativement le cube dudit diamètre.

3. Appareil selon la revendication 1, **caractérisé en ce que** ledit axe de rotation (100) dudit récipient (10) est inclinée à un angle égal à approximativement 45° par rapport à la ligne droite que trace la distance la plus courte entre les génératrices desdits corps périphériques (13, 14) qui sont parallèles à au moins une génératrice dudit corps central (12) .

4. Appareil selon la revendication 1, **caractérisé en ce qu'**il comprend au moins un dispositif d'anti-agrégation (19) agencé à l'intérieur dudit récipient stérilisable (10); ledit dispositif d'anti-agrégation (19) comprenant une ou plusieurs pales (20, 21) qui sont associées de manière amovible aux parois internes desdits corps périphériques (13, 14); chacune desdites pales (20, 21) comportant une pointe (22, 23) dirigée en direction de la face extrémité la plus petite du corps périphérique (13) qui est situé à l'opposé de celui avec lequel il est associé.

5. Appareil selon la revendication 4, caractérisé en ce une ou plusieurs pales (20, 21) sont associées de manière amovible aux parois internes desdits corps périphériques (13), avec la possibilité de régler la position et l'inclinaison; chacune desdites pales (20, 21) étant inclinée à un angle compris entre approximativement 10° et approximativement 90° par rapport aux génératrices dudit corps central (12).

6. Appareil selon la revendication 4, **caractérisé en ce que** lesdites une ou plusieurs pales (20, 21) qui sont associées de manière amovible aux parois internes desdits corps périphériques (13), avec la possibilité de régler la position et l'inclinaison; chacune desdites pales (20, 21) étant inclinée à un angle compris entre approximativement 20° et approximativement 25°, par rapport aux génératrices dudit corps centrale (12).

7. Appareil selon la revendication 1, **caractérisé en ce que** lesdits moyens de traitement aval comprennent un ou plusieurs dispositifs de fraisage associés de manière amovible audit récipient (10), un ou plusieurs dispositifs d'homogénéisation associés de manière amovible audit récipient (10), un ou plusieurs dispositifs d'extraction associés de manière amovible audit récipient (10), et une plusieurs soupapes de décharge pour des solides ou des liquides; lesdits moyens de traitement aval étant agencés essentiellement au niveau des faces d'extrémité plus petites desdits corps périphériques (13) et étant associables avec ledit récipient (10) par lesdits moyens de couplage.

8. Appareil selon la revendication 1, **caractérisé en ce que** ledit récipient (10) est associé de manière rotative à ladite structure de support (2) au moyen d'au moins un arbre d'essieu creux (5), qui est relié de manière fonctionnelle à un moyen d'actionnement rotatif (7, 8); ledit moyen d'actionnement rotatif (7, 8) étant commandé par lesdits moyens de contrôle de fermentation.

9. Appareil selon la revendication 8, **caractérisé en ce que** ledit récipient (10) comporte une veste (26) dans laquelle un fluide circule à une température contrôlée permettant une commande thermostatique du récipient (10).

10. Appareil selon la revendication 1, **caractérisé en ce que** lesdits moyens de contrôle de fermentation comprennent une pluralité de capteurs permettant de détecter les paramètres de processus associés audit récipient (10); au moins une partie desdits capteurs étant reliée de manière fonctionnelle à un moyen de transmission de données.

11. Appareil selon la revendication 8 et 11, **caractérisé en ce que** lesdits moyens de contrôle de fermentation comprennent au moins une unité électronique équipée d'un moyen d'interface utilisateur destiné à une commande sur site et à distance; ladite au moins une unité électronique étant reliée de manière fonctionnelle auxdits capteurs, audit moyen de traitement aval et audit moyen d'actionnement rotatif.
